Europäisches Patentamt

European Patent Office

Office européen des brevets

(19)

(11) **EP 1 171 146 B1**

(12) # EUROPEAN PATENT SPECIFICATION

(45) Date of publication and mention
of the grant of the patent:
**23.06.2004 Bulletin 2004/26**

(21) Application number: **99916052.6**

(22) Date of filing: **21.04.1999**

(51) Int Cl.7: **A61K 35/78**, A61K 31/135,
A61P 25/16, A61P 25/28

(86) International application number:
**PCT/KR1999/000189**

(87) International publication number:
**WO 2000/064462 (02.11.2000 Gazette 2000/44)**

(54) **PHARMACEUTICAL COMPOSITIONS CONTAINING SELEGILINE AND GINKGO BILOBA EXTRACT USEFUL FOR TREATING DEMENTIA**

PHARMAZEUTISCHE ZUSAMMENSETZUNGEN DIE SELEGILIN UND GINKGO BILOBA EXTRAKT ENTHALTEN ZUR BEHANDLUNG VON DEMENZ

COMPOSITIONS PHARMACEUTIQUES CONTENANT DE LA SELEGILINE ET DE L'EXTRAIT DE GINKGO BILOBA POUR TRAITER LA DEMENCE

(84) Designated Contracting States:
**DE FR GB**

(43) Date of publication of application:
**16.01.2002 Bulletin 2002/03**

(73) Proprietor: **Yuyu Inc.**
**Anyang-si, Kyungki-do 430-040 (KR)**

(72) Inventors:
• **Ann, Hyung Soo**
**Seoul 139-230 (KR)**
• **Lee, Jeong Mi**
**Seoul 138-225 (KR)**
• **Kim, Hyung Chun**
**Kangwon-do 200-030 (KR)**

• **Kang, Sung An**
**Seoul 137-049 (KR)**

(74) Representative: **Smart, Peter John**
**W.H. BECK, GREENER & CO**
**7 Stone Buildings**
**Lincoln's Inn**
**London WC2A 3SZ (GB)**

(56) References cited:
**WO-A1-96/19981          WO-A1-96/26720**

• **PATENT ABSTRACTS OF JAPAN vol. 199, no. 803 27 February 1998 & JP 09 301 857 A (FUJIMOTO SEIYAKU KK) 25 November 1997**

## Description

[FIELD OF THE INVENTION]

This invention relates to pharmaceutical compositions containing selegiline and Ginkgo biloba extract as their active components and being useful for treatment of parkinson's disease and improvement of symptoms of dementia.

[DESCRIPTION OF THE PRIOR ART]

Selegiline(known also as "deprenyl") is clinically used for Parkinson's disease as it indirectly increases dopamines by selectively inhibiting Monoamine oxidase B ($MAO_B$).[Arch. Gen. Pyschiatry, 44, 427(1987)]. Moreover, there are some clinical reports stating that it is also effective in Alzheimer's disease as it exerts protective effects for neuronal cells thanks to its antioxidant effects as well as possessing antidepressant effects through inhibition of reabsorption of Monoamine oxidase. [Arch. Gen.Psychiatry, 46, 45(1989), Ann. Neural., 26, 689(1989), Arch. Gen.Psychiatry, 44, 427(1987)].

On the other hand, Ginkgo biloba extract is an extract obtained by extracting the leaves of Ginkgo biloba Linne with acetone and water and it contains 24 % of flavonoids or Ginkgo-flavone glycosides and 6 % of terpenoids or Ginkgolides or bilobalides as the representative components. Besides, it also includes proanthocyanidin and organic acids, etc. [Vanhaelen, Planta Med., 55, 202(1989)].

Flavonoids are free radical scavengers and exert anti-oxidant effects while Ginkgolide B inhibits aggregation of platelets as it possesses PAF inhibitory action.[Eur. J. Pharmacol., 127(1-2), 83. 1986]

Ginkgo biloba extract ameliorates circulatory disturbances of microvessels through combined action rather than through any individual action of such various active components and is used for treatment of brain dysfunction accompanying dementia-like symptoms including such as tinnitus, headache, loss of memory, concentration disturbance and depression, etc.

[SUMMARY OF THE INVENTION]

From the above, combined administration of selegiline and Ginkgo biloba extract to a patient with dementia is expected to produce synergistic effects of two different action mechanisms of antidepressant effects and blood flow increasing effects and to potentiate the protective effects for neuronal cells, which are common to both components. Thus, the purpose of the present invention is to provide pharmaceutical compositions expected capable to exert effects for improvement of symptoms of parkinson's disease and dementia.

This invention relates to pharmaceutical compositions contaninig selegiline and Ginkgo biloba extract and being useful for treatment of symptoms of parkinson's disease and dementia, in which the combination ratios of selegiline and Ginkgo biloa extract ranges between 1:15 and 1:30 by weight, preferably containing 2-5 mg of selegiline and 30-150 mg of Ginkgo biloba extract per doasage form.

**[BRIEF DESCRIPTION OF THE DRAWINGS]**

Figure 1 represents the effects of single or combined 3-week administration of selegiline(2 mg/kg/day, p.o.) and Ginkgo biloba extract(48 mg/kg/day, p.o.) in the passive avoidance test done on rats injected with scopolamine(6 mg/kg, **s. c.** ).
(* p<0.05, ** p<0.01: Significantly different from the control)
Figure 2 represents the effects of single or combined 3-week administration of selegiline(2 mg/kg/day, p.o.) and Ginkgo biloba extract(48 mg/kg/day, p.o.) on the number of visit errors committed by rats put on the eight-arm radial maze test after 3-week pretreatment with the drug(s).
Figure 3 represents the effects of single or combined 3-week administration of selegiline(2 mg/kg/day, p.o.) and Ginkgo biloba extract(48 mg/kg/day, p.o.) on the levels of dopamine and its metabolites in the cerebral cortex of ischaemic rats.
(* p<0.05, *** p<0.001: Significantly different from the control)
Figure 4 represents a set of photographs of neuronal degenerations shown by cresyl-violet staining of the hippocampus of Mongolian gerbil reperfused following induced ischemia after 3-week pretreatment with the drug(s), in which:
4a: pyramidal neuronal cells at CA1 (Ammon's horn) area of the hippocampus of normal gerbil shown at lower magnification ($\times$40).

4b: pyramidal neuronal cells at CA1 (Ammon's horn) area of the hippocampus of normal gerbil shown at higher magnification ($\times$200).

4c: pyramidal neuronal cells at CA1 (Ammon's horn) area of the hippocampus of control gerbil reperfused for 7 days after ischemia induced by ligation of carotid artery, shown at lower magnification($\times$40).

4d: pyramidal neuronal cells at CA1 (Ammon's horn) area of the hippocampus of control gerbil reperfused for 7 days after ischemia induced by ligation of carotid artery, shown at higher magnification ($\times$200).

4e: pyramidal neuronal cells at CAl(Ammon's horn) area of the hippocampus of gerbils received single 3-week administration of selegiline, shown at lower magni-fication($\times$40).

4f: pyramidal neuronal cells at CAl(Ammon's horn) area of the hippocampus of gerbils received single 3-week administration of selegiline, shown at higher magni-fication ($\times$200).

4g: pyramidal neuronal cells at CAl(Ammon's horn) area of the hippocampus of gerbils subjected to single 3-week administration of Ginkgo biloba extract, shown at lower magnification($\times$40).

4h: pyramidal neuronal cells at CA1(Ammon's horn) area of the hippocampus of gerbils subjected to single 3-week administration of Ginkgo biloba extract,shown at higher magnification($\times$200).

4i: pyramidal neuronal cells at CA1(Ammon's horn) area of the hippocampus of gerbils subjected to combined 3-week administration of selegiline and Ginkgo biloba extract, shown at lower magnification($\times$40).

4j: pyramidal neuronal cells at CA1 (Ammon's horn) area of the hippocampus of gerbils subjected to combined 3-week administration of selegiline and Ginkgo biloba extract,shown at higher magnification( X200).

## [DETAILED DESCRIPTION OF THE INVENTION]

Pharmaceutical compositions of the present invention being capable to improve symptoms of parkinson's disease and dementia comprise pharmaceutically effective doses of selegiline and Ginkgo biloba extract and the ratio of combination ranges from 1:15 to 1:30 by weight. More preferable are combinations of selegiline between 2.0 and 5.0 mg and Ginkgo biloba extract between 30 and 150 mg per dosage form.

Pharmaceutical compositions of the present invention contains, in addition to the above components, one or more pharmaceutically acceptable components selected from a group consiting of excipients, disintegrating agents, binders, and lubricating agents, etc. As for pharmaceutical dosage forms, film-coated tablets, hard gelatin capsules or granules are desirable. Each film-coated tablet contains 5 mg of selegiline hydrochloride and 120 mg of Ginkgo biloba extract and is orally administered one to two times a day depending on the severity of symptoms and the dosage should be properly reduced to a patient with renal deficiency, in particular.

Embodiments of the present invention are illustrated in the following examples of practice and experiments.

(Examples)

(Formulation example 1)

film-coated tablet

Preparation: In accordance with the usual known methods in the pharmaceutical manufacturing technology, weighed amounts of both active and auxiliary components of the present invention as shown below are mixed in a ribbon-type mixer and then put into a compress granulator to obtain granules.

After drying at 50°C, the granules are compressed into tablets by using rotary tableting machine and the tablets are film-coated according to conventional pharmaceutical methods. This film-coated tablet(420 mg) contains 120 mg of Ginkgo biloba extract and 5 mg of selegiline hydrochloride per tablet.

Composition

Each tablet (420 mg) contains:

| Active ingredient | Ginkgo biloba extract (Notice No.96-2, Suppl.6, Korea Food and Drug Administration Office) (Equivalent to 28.8 mg as total Ginkgo flavonoid glycosides) | 120.000 mg |
|---|---|---|
| | Selegiline hydrochloride | 5.000 mg |
| Excipients | Lactose(K.P.) | 63.760 mg |
| | Microcrystalline cellulose(NF) | 95.125 mg |
| | Corn starch(K.P.) | 48.000 mg |

(continued)

| | | |
|---|---|---|
| | Colloidal silicate anhydrous(NF) | 40.500 mg |
| Disintegrating Agent | Croscarmellose sodium(NF) | 15.750 mg |
| Lubricating Agent | Magnesium stearate(K.P. ) | 1.875 mg |
| Coating Agent | Hydroxymethylcellulose 2910(K.P.) | 17.340 mg |
| | Polyethyleneglycol 4000(K.P.) | 8.675 mg |
| | Talc(K.P.) | 1.080 mg |
| | Simethicone Emulsion(USP) | 0.015 mg |
| Sunscreen Agent | Titanium oxi de(K. P. ) | q. S. |
| Coloring Agent | Ferric oxide(NF) | q.s. |
| Glossing Agent | Carnauba wax(K.P.) | q. s. |

**(Formulation Example 2)**

**Hard gelatine capsule**

Preparation: In accordance with the usual known methods in the pharmaceutical manufacturing technology, weighed amounts of both active and auxiliary components of the present invention as shown below are mixed in a ribbon-type mixer and then put into a compress granulator to obtain granules.
After drying at 50°C, the granules are filled into capsules of size number 2 in weight of 250 mg per capsule. This capsule contains 120 mg of Ginkgo biloba extract and 5 mg of selegiline hydrochloride per capsule.

Composition:

Each capsule (250 mg) contains:

| | | |
|---|---|---|
| Active ingredient | Ginkgo biloba extract (Notice No. 96-2, Suppl.6, Korea Food and Drug Administration Office) (Equivalent to 28.8 mg as total Ginkgo flavonoid glycosides) | 120.000 mg |
| | Selegiline hydrochloride | 5.000 mg |
| Excipients | Lactose(K.P.) | 115.000 mg |
| Coating Agent | Magnesium stearate(K.P.) | 10.000 mg |
| Capsule | Yellow in the top and light yellow | |
| | in the bottom(K.P. ) | q.s. |

(Formulation Example 3)

Granules

Preparation: In accordance with the usual known methods in the pharmaceutical manufacturing technology, weighed amounts of both active and auxiliary components of the present invention as shown below are mixed in a ribbon-type mixer and then put into a compress granulator to obtain granules.
After drying at 50°C, the granules are dispensed into stick-type sachets in weight of 1,000 mg per pack. This sachet contains 120 mg of Ginkgo biloba extract and 5 mg of selegiline hydrochloride per pack.

Composition:

Each capsule (1,000 mg) contains:

| | | |
|---|---|---|
| Active ingredient | Ginkgo biloba extract (Notice No.96-2, Suppl.6, Korea Food and Drug Administration Office) (Equivalent to 28.8 mg as total Ginkgo flavonoid glycosides) | 120.000 mg |
| | Selegiline hydrochloride | 5.000 mg |
| Excipient | Lactose(K.P.) | 350.000 mg |

(continued)

| | | |
|---|---|---|
| | Carboxymethylcellulose(K.P. | 8.50 mg |
| | Corn starch(K.P.) | 495.000 mg |
| | Colloidal silicate | 20.500 mg |
| anhydrous(NF) | | |
| Talc(K.P.) | | 1.000 mg |

(Experiment Examples)

(Experiment Example 1)

Stability test(six months, at room temperature and at 40°C, 75 % RH, Accelerated short term test)

For the tablets prepared in the Formulation Example 1 above, a stability test was carried out and the test results are shown in Table 1, Table 2 and Table 3.

Analytical test methods

1)Appearance: Observed with eyes for any visible defects.

2)Assay:

① Selegiline (HPLC Method)

Put more than 20 of the tablets in the Formulation Example 1 above into a mortar, powderize, weigh and take accurately the amount of powder equivalent to 50 mg of selegiline into a 50-m$\ell$ volumetric flask. Add 40 m$\ell$ of the mobile phase solvent, extract for 10 minutes under ultrasonic stirring and fill to the mark.

After taking 25 m$\ell$ of this solution into a centrifuge tube, centrifuge for 10 minutes at 3,500 rpm and then transfer accurately 10 m$\ell$ of the supernatant into a 100-m$\ell$ volumetric flask, add the mobile phase solvent to the mark of 100-m$\ell$ and use as test solution.

Seperately, weigh and take precisely 50 mg of selegiline reference standard into a 50-m$\ell$ volumetric flask, dissolve by adding the mobile phase solvent to the mark. Transfer 10 m$\ell$ of this solution into a 100 m$\ell$ volumetric flask, add the mobile phase solvent to the mark and use as standard solution.

For 20 $\mu\ell$ each of the test and the standard solutions, perform liquid chromatography at the following conditions and determine the respective areas under the peaks AT and AS according to the given formula.

$$\text{Selegiline(mg)= 500 C(AT/AS)}$$

Where:

C: Concentration of standard solution(mg/m$\ell$)
AT: Area under the peak of test solution
AS: Area under the peak of standard solution

**Operating conditions for HPLC**

Detector:        UV Absorption Spectrophotometer (wave length 205 nm)
Column:         C8 column with inner diameter of 3.9 mm and in length of 30 cm.
Mobile Phase:   A 80:20 mixture of 0.1 M Ammonium phosphate
(pH 3.1):        Acetonitrile
Flow rate:       1.0 m$\ell$/min

② Assay for Ginkgo flavon glycoside (HPLC Method)

Put more than 20 of the tablets in the Formulation Example 1 above into a mortar, powderize, weigh and take accurately the amount of powder equivalent to 150 mg of Ginkgo biloba extract into a 50-mℓ volumetric flask. Add 30 mℓ of methanol, extract for 10 minutes under ultrasonic stirring and fill to the mark.

Afrer taking 25 mℓ of this solution into a centrifuge tube, centrifuge for 10 minutes at 3,500 rpm and transfer accurately 10 mℓ of the supernatant into a 30-mℓ test tube. Mix well after adding 10 mℓ of 1.5 N hydrochloric acid, tightly close the test tube and place and keep it in a water bath of 90-100°C in temperature for 25 minutes to complete hydrolysis. Thereafter, rapidly cool and filter the solution and use the filtrate as test solution.

Separately, weigh precisely 5.0 mg each of respective reference standards of Quercetin, Kaempferol, and Isorhamnetin and place them individually into 50-mℓ flasks, dissolve by adding methanol to the 50-mℓ mark line and use as respective standard solutions.

Perform liquid chromatographies for 20 μℓ each of both standard and test solutions according to the operating conditions specified below, measure respective areas under the peaks and determine the contents of each component with the formula given below.

Total of Ginkgo flavonoid glycosides(mg) = Quercetin

glycoside(mg) + Kaempferol glycoside(mg) + Isorhamnetin

glycoside(mg)

Operating conditions for HPLC

Detector:        UV Absorption Spectrophotometer (wave length 365 nm)
Column:        Nova pak C18 column
Mobile phase:    water: methanol : glacial acetic acid = 60:40:3
Flow rate:        1.0 mℓ/min

Results of Experiment 1

As shown in Table 1, Table 2 and Table 3 below, three lots of Formulation Example 1 were prepared, filled tightly into ampoules and stored for 6 months at room temperature or at 45 °C with 75 % RH and then subjected to the analytical tests.

No changes of any significance in appearance, content or property were observed and the product was stable. So, it was conceived that the product would be good for 24 month expiry.

[Table 1 ]

| Storage | Test Item | Standard | Initial | Month 2 | Month 4 | Month 6 |
|---|---|---|---|---|---|---|
| Room Temp. | Appearance | Yellow, rectangular, film-coated tablet | Yes | Yes | Yes | Yes |
| | Moisture | <5% | 3.11 | 3.29 | 3.44 | 3.43 |
| | Disintegration | <30 min | 11-13 | 11-13 | 11-13 | 11-13 |
| | Selegiline | 5 mg | 5.2 | 5.3 | 5.2 | 5.3 |
| | Ginkgo flavon glycoside | 28.8 mg | 30.8 | 30.3 | 31.6 | 30.5 |
| | Terpene lactone | 7. 2 mg | 7.7 | 7.7 | 7.8 | 7.7 |

[Table 1 ]　(continued)

| Storage | Test Item | Standard | Initial | Month 2 | Month 4 | Month 6 |
|---|---|---|---|---|---|---|
| 40 °C 75 % RH | Appearance | Yellow, rectangular, film-coated tablet | Yes | Yes | Yes | Yes |
| | Moisture | <5% | 3.11 | 3.64 | 3.64 | 3.73 |
| | Disintegration | <30 min | 11-13 | 12-14 | 11-14 | 12-14 |
| | Selegiline | 5 mg | 5.2 | 5.2 | 5.3 | 5.2 |
| | Ginkgo flavon glycoside | 28.8 mg | 30.8 | 31,6 | 30.3 | 30.9 |
| | Terpene lactone | 7.2 mg | 7.7 | 7.8 | 7.7 | 7.6 |

[Table 2]

| Storage | Test Item | Standard | Initial | Month 2 | Month 4 | Month 6 |
|---|---|---|---|---|---|---|
| Room Temp. | Appearance Appearance | Yellow. rectangular, film-coated tablet | Yes Yes | Yes Yes | Yes Yes | Yes Yes |
| | Moisture | <5 % | 3.19 | 3.17 | 3.36 | 3. 69 |
| | Disintegration | <30 min | 11-13 | 11-13 | 11-13 | 11-12 |
| | Selegiline | 5 mg | 5. 2 | 5.3 | 5.2 | 5.3 |
| | Ginkgo flavon glycoside | 28.8 mg | 31.6 | 31.3 | 32. 2 | 31.5 |
| | Terpene lactone | 7. 2 mg | 7.4 | 7.3 | 7.2 | 7.3 |
| 40°C 75 % RH | Appearance | Yellow. rectangular, film-coated tablet | Yes | Yes | Yes | Yes |
| | Moisture | <5% | 3.19 | 3.64 | 3.64 | 3.73 |
| | Disintegration | <30 min | 11-13 | 12-15 | 11-14 | 11-16 |
| | Selegiline | 5 mg | 5.2 | 5.3 | 5.3 | 5.2 |
| | Ginkgo flavon glycoside | 28.8 mg | 31.6 | 31.9 | 30.8 | 31.3 |
| | Terpene lactone | 7.2mg | 7.4 | 7.3 | 7.5 | 7.4 |

[Tabl e 3]

| Storage | Test Item | Standard | Initial | Month 2 2 | Month 4 4 | Month 6 |
|---|---|---|---|---|---|---|
| Room Room Temp. | Appearance Appearance | Yellow, rectangular, film-coated tablet | Yes Yes | Yes Yes | Yes Yes | Yes Yes |
| | Moisture | <5 % | 3.07 | 3.16 | 3.14 | 3.13 |
| | Disintegration | <30 min | 11-16 | 11-15 | 11-13 | 11-15 |
| | Selegiline | 5 mg | 5.3 | 5.3 | 5.2 | 5.2 |
| | Ginkgo flavon glycoside | 28. 8 mg | 32. 0 | 30. 3 | 31.6 | 30.5 |
| | Terpene lactone | 7.2 mg | 7.6 | 7.9 | 7.9 | 7.7 |

[Tabl e 3]   (continued)

| Storage | Test Item | Standard | Initial | Month 2 2 | Month 4 4 | Month 6 |
|---|---|---|---|---|---|---|
| 40°C 75% RH | Appearance | Yellow, rectangular, film-coated tablet | Yes | Yes | Yes | Yes |
| | Moi sture | <5% | 3. 07 | 3.64 | 3.64 | 3.73 |
| | Disintegration | <30 min | 11-16 | 12-14 | 11-14 | 12-17 |
| | Selegiline | 5 mg | 5.3 | 5.2 | 5.3 | 5.1 |
| | Ginkgo flavon glycoside | 28.8 mg 28.8 mg | 32.0 32.0 | 31.6 31.6 | 30.3 30.3 | 30.9 30.9 |
| | Terpene lactone | 7.2 mg | 7.6 | 7.6 | 7.5 | 7.5 |

(Experiment Example 2)

Experiment on the effects of combined administration of selegiline and Ginkgo biloba extract on improvement of symptoms of dementia

I)Foreword

As part of a plan to develop the combination formula of 5 mg of selegiline and 120 mg of Ginkgo biloba extract (1:24), based on the respective clinical dosage in practice, as a drug for senile dementia, we have carried out preclinical evaluations on the effects of the combination formula in comparison with the respective single drugs.
Behavioral pharmacological evaluations were made for improvement in memory and learning functions in rats with memory loss induced by scopolamine. At the same time, as the major pathology of senile dementia is known to be closely related with Alzheimer's disease caused by denatured proteins in the neuronal cells and also with neuronal damages caused by ischemia, studies on biochemical changes in the cerebellum and histopathological examinations of the hippocampus, a site related to memory function, were made in ischemic rats.

2)Mehods of Experiment

(1)Animals and materials

Male Sprague-Dawley rats with body weights of 200-300 g and male gerbil were used. Feeds were freely supplied and drugs were given dissolved in water supply. Environmental controls were maintained at temperature of 23±1 °C and relative humidity of 55±5 % and with the darkness-light alternating at 12 hour intervals.
Animals are classified into four groups, namely, a normal group, a selegiline group at dose of 2 mg/kg/day, a Ginkgo biloba extract group at dose of 48 mg/kg/day and a group taking combination formula of selegiline 2 mg/kg/day and Ginkgo biloba extract 48 mg/kg/day and drugs were administered orally for three weeks.
Selegiline(Lot No. 9706005) was supplied by Sanofi S.A., France and Ginkgo biloba extract(Lot No. 708601-1) was by Yuyu Industrial Co., ltd.

(2)Methods

① Behavioral pharmacology experiment

To evaluate effects on memory and learning functions, rats of each group were challenged with injection of scopolamine, a drug which cause memory loss and then, subjected to the following tests.

(i)Passive avoidance response test

Rats of each group were previously subjected to the adaptation training and subsequent exercises in a shuttle box of step-through type. In the real experiment, scopolamine at a dose of 6 mg/kg was given intraperitoneally and, after 30 minutes, passive avoidance responses were determined. Longer latency in response time indicate improved memory and learning fuctions.

(ii)Maze test

According to 01 ton and Samuelson methods[J. Exp. Psychol. Anim. Behav. Proc. 2, 97(1976)], 8-arm radial maze test system was used. That is, rats of each group were placed in the center of the eight 65-cm arms of the maze test system, which are radially stretched, and 45 mg each of pellet type feed was placed at the end of each arm. Rats were trained to visit the arm-ends to get the pellet type feeds. In real experiment, rats were pre-treated with intraperitoneal injection of scopolamine at a dose of 6 mg/kg as a challenge and subjected to the test after 30 minutes from injection.

Test was conducted for ten minutes and numbers of arms failed to receive visit and numbers of arms which received repeated visits were counted and recorded collectively as visit errors. Test was repeated for fifty(50) times. Smaller visit errors indicate gained improvements in memory and learning functions.

② Changes in dopamine and energy metabolism in partial ischemia

Rats of each group were operated under urethane anesthesia to perform ligation of the carotid artery for 15 minutes to induce partial ischemia to the cerebellum. Then, the cerebellum was immediately cut out and stored under refrigeration by liquid nitrogen.

(i)Measurement of dopamine and its metabolites concentrations

To 0.1 g of cerebral cortex taken from the cerebellum, 0.1 m$\ell$ of ascorbate oxidase at concentration of 1.0 mg/m$\ell$ and 1.9 m$\ell$ of mixture of 0.1 M acetic acid and 1.0 mM glutathione were added and homogenized. Then, 100 $\mu\ell$ aliquots of the homogenate sample were transferred into two Ependorff tubes and protein concentration was measured with one tube.

To the remaining tube, 2.0m$\ell$ (or in a volume of the same proportion to the amount of sample) of 0.1 M perchloric acid and 2 ng/50 $\mu$l DHBA(3,4-dihydroxybenzylamine) were added and homogenized. Then, the solution was centrifuged for 20 minutes at 11,000 rpm, the supernatant was taken and filtered through 0.2 $\mu$m membrane filter (MFS-13 CA membrane) and 15 $\mu\ell$ of the filtrate was injected into HPLC system.

The HPLC system used was of Waters & Co., USA and as for electro-chemical detector, Ag/AgCl reference electrode and single glassy carbon working electrode were used. The electric current (1 range) was set at 10 nA, time constant at 1.0 second and E(potential) at +0.75 V, respectively.

For preparation of mobile phase solution, EDTA. 2Na 29.2 mg, sodium phosphate 21.9 g and octanesulfonic acid 368 mg were dissolved into one liter of distilled water and pH was adjusted at 3.5. Then, 900 m$\ell$ of this solution was mixed with 100 m$\ell$ of methanol 1 and filtered through 0.2 $\mu$m membrane filter. The solution was then sonicated to remove air bubbles and used.

As for column, $\mu$-Novapac C18 reverse phase column was used and the flow rate was set at 0.6 m$\ell$/min. Assay for each component was made by comparing areas under the peaks for standard and test samples. As for standards, DOPAC(3,4-dihydroxyphenylacetic acid), DA(Dopamine) and HVA( Homovanillic acid) were dissolved in a solution of 0.1 M perchloric acid and 0.05 mM glutathione to a final concentration of 20 ng/m$\ell$ and 15$\mu\ell$ each was injected.

(ii)Measurement of energy metabolites in the cerebral cortex

The cerebellum obtained as above was incised and added with a mixed solution of 0.3 M HC10$_4$ and 0.25 mM EDTA in an amount of 4 times the weight of the tissue, kept still at room temperature for 10 minutes, homogenized and again kept still for 15 minutes and then neutralized with 2.5 M K$_2$CO$_3$. After being centrifuged for 20 minutes at 1,000 g, it was filtered through 0.45 $\mu$m filter and used as sample.

Using reagent kits, concentrations of glucose, pyruvate, lactate and ATP were determined by a spectrophotometer.

③ Antioxidant effects in partial ischemia and reperfusion

Rats of each group were anesthetized by injecting pentobarbital sodium(30 mg/kg, i.p.) and ketamine(5 mg/kg, s.c.) and were operated to expose the whole carotid artery. Then, the carotid artery was ligated to induce ischemia for 30 minutes, thereby, blocking blood flow to the brain, followed by subsequent reperfusion for 3 hours and then, the cerebral cortex was excised and antioxidant effects were measured.

(i) Measurement of Malondialdehyde

After adding 1.5 M Potassium Chloride solution to the excised cerebral cortex in a volume ratio of 9 m$\ell$ per each gram of the tissue, the tissue was homogenized and centrifuged. (3,000 rpm, 15 min, 4 °C).

Then, 1.0 m$\ell$ of the supernatant was transferred into a test tube containing 2.0 m$\ell$ of TBA(Tiobarbituric acid) reagent, mixed with vortex shaking and boiled in a thermostat of 100°C for 15 minutes after placing a glass ball on the mouth of the pyrex test tube.

After 15 minutes, the contents are immediately cooled by applying to a flowing water or soaking into ice water and centrifuged. (3,000 rpm. 15 min, 4 °C). The supernatant was tested for absorbancy at 535 nm using TBA(Tiobarbituric acid) reagent as blank. [Gerontology, 43, 218(1987)]

(ii) Measurement of SOD( Superoxide dismutase ) activity

After adding 1.5 M Potassium Chloride solution to the excise cerebral cortex in a volume ratio of 9 m$\ell$ per gram, the tissue was homogenized and centrifuged.(3,000 rpm, 15 min. 4 °C). The supernatant was used as sample.

Previously, a test tube containing 2.3 m$\ell$ of phosphate buffer with pH 7.8, 300 $\mu\ell$ of 0.5 mM Na-xanthine, 100 $\mu\ell$ of 50 $\mu$m K-cyanide and 100 $\mu\ell$ of 2.4 mM Na-deoxycholate was prepared and kept in a water bath of 25 °C. To this test tube, 300 $\mu\ell$ of 0.1 mM ferricytochrome and sample were added.

Then. the solution was transferred to a cell, 100 $\mu\ell$ of xanthine oxidase was added, a sealing tape was placed on the cell and, while closing well with fingers, the cell was carefully shaken to mix. Changes in absorbance were measured and recorded for one minute at the wavelength of 550 nm.

(iii) Measurement of glutathione(GSH) peroxidase activity

After adding 1.5 M Potassium Chloride solution to the excise cerebral cortex in a volume ratio of 9 m$\ell$ per each gram of the tissue, the tissue was homogenized and centrifuged.(3,000 rpm, 15 min, 4°C. The supernatant was used as sample. To a test tube containing 1.0 m$\ell$ of phosphate buffer with pH 7.0, 0.2 m$\ell$ of 0.01 M NaN$_3$,0.2 m$\ell$ of 0.01 M GSH(glutathione), 0.2 m$\ell$ of 1.5mM NADPH (Nicotinamide adenine dinucleotide phosphate) and 0.2 me of 0.5 % glutathione reductase, 0.2 m$\ell$ of sample and 1.0 m$\ell$ of distilled water were added to a final volume of 3.0 m$\ell$.

After mixing well with vortex shaking, the test tube was kept standing for 5 minutes at 25°C and then 0.1 m$\ell$ of 5.0 mM H$_2$O$_2$ was added to initiate reaction. Initial absorbance was taken at 340 nm and, then in sequence, absorbances were measured each minute for 3 times and calculations were made according to the following formula.

$$\Delta C(\text{nmol/min}) = (\Delta OD/\varepsilon) \times 10^3$$

where:

C: Enzyme activity expressed in units.
One unit is equivalent to the amount of enzyme capable to consume one nmol of NADPH per minute.
OD: Optical density
$\varepsilon$: Molar absorbance constant: $6.22 \times 10^3 M^{-1}$ cm$^{-1}$

④ Histopathological examination of hippocampus after complete ischemia and reperfusion

Gerbils of both control and test groups were anesthetized with injection of pentobarbital sodium at a dose of 50 mg/kg and the carotid arteries on both sides were closed for 5 minutes to induce complete ischemia. After 7 days from initiation of reperfusion, tissues were examined by means of cresyl-violet staining.

Tissue examination under cresyl-violet staining mentioned above was done as follows. Excised cerebellum was cut into slices using sliding microtome and the slices were treated sequentially with xylene and alcohol for five minutes and finally, soaked into distilled water for another 5 minutes. Then, the slice was stained with cresyl-violet which had been prepared in advance.

After staining, the slice was washed one time for 30 minutes with flowing water, followed by dehydrating in alcohol and subsequent two time washings with xylene for 10 minutes. Then, a cover glass was placed on and the specimen was examined under optical microscope.

* Statistical Treatment

The test results were expressed in the form of mean±S.E. and statistical significance was evaluated by student's t-test.

3)Results and Discussion

(1) Effects in Behavioral Pharmacology

① Passive avoidance response test

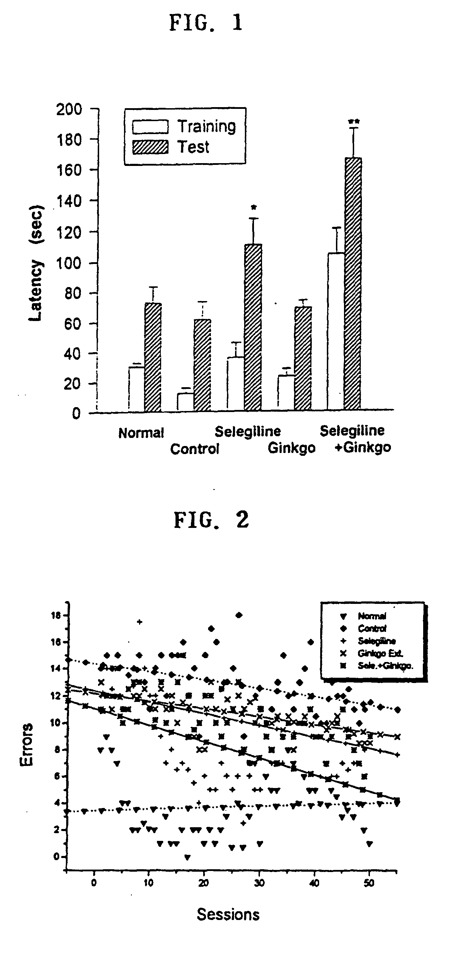

In a step-through passive avoidance test, rats of both normal and test group treated with drugs for 3 weeks were put in a shuttle box and tested for latency time required for avoidance response after being given with scopolamine injection to induce memory loss and the results are shown in Figure 1.
In contrast to normal group, a significant increase in the response time($p<0.05$) was obtained in the selegiline group given with the single drug and a more significant increase($p<0.01$) was seen with the group given with both selegiline and Ginkgo biliba extract in combination.
Such increases in the avoidance response time suggest increased capabilities in memory and learning functions. In case of the group given singly with Ginkgo biloba extract, no significant increase in the response time was observed. But, when selegiline was given in combination with the extract, it tended to increase the response time much more than that by selegiline alone. This result is in good agreement with the previous report stating that a remarkable increase in memory function was a result from the increased dopamine level induced by inhibition of monoamine oxidase B by selegiline.

[Psychopharmacology, 91, 489(1987): The neurosciences second study program, 324(1970)]

② Maze test

In order to induce memory loss, scopolamine was injected to the rats of both normal and test groups which received drug treatment for 3 weeks and then the rats were placed in the center of the 8-arm maze test system to record numbers of visit errors to the respective arms. The test results are shown in Figure 2.
In comparison to normal group, there was an apparent increase in visit errors in rats of control group treated with scopolamine.
In both selegiline-alone and Ginkgo biliba extract-alone groups, the visit errors tended to decrease at a similar degree when compared with control group. In case of the group given with selegiline and Ginkgo biloba extract in combination, the visit errors got much smaller than those in groups treated with the respective single drug and, as the experiment continued, learning capability was gradually increased and a test result similar with normal group was obtained.
This result is very similar with the previous report by Blavet et al who reported improvement in learning function upon maze test in a rat treated with Ginkgo biliba extract at a dose of 30 mg/kg/day.[Effect of Ginkgo biloba extract (EGb 761) on the Central Nervous System, christien,Y, eds, Elsevier (Paris). 119 (1992)].

(2)Changes in dopamine and energy metabolism in partial ischemia

① Measurement of dopamine and its metabolites

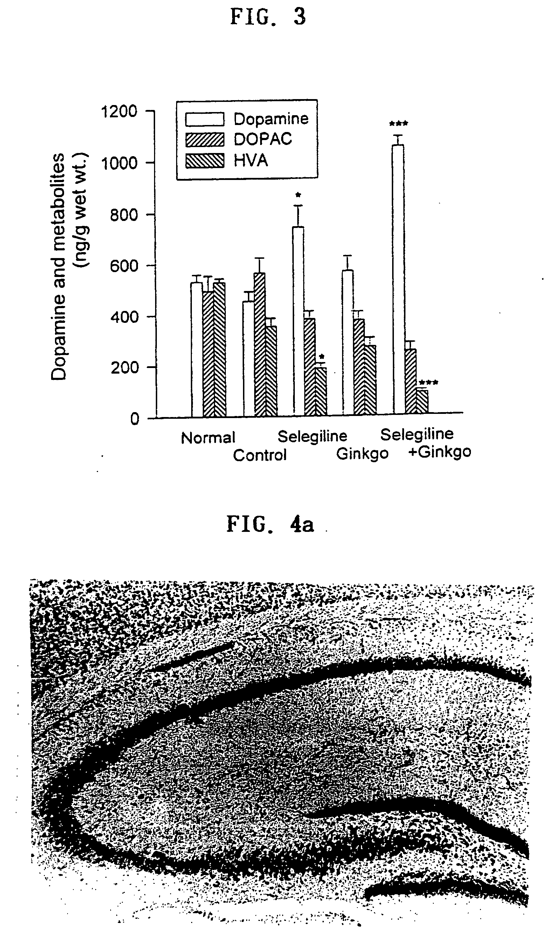

Partial ischemia was induced in rats of both normal and treatment groups to cause brain damage which is said to be related with dementia. Concentrations of dopamine and its metabolites in the cerebral cortex were measured and the results are shown in Figure 3.
In both selegiline-alone group and selegiline-Ginkgo biloba extract combination group, significant increases in content of dopamine were observed($p<0.05$ & $p<0.001$ respectively) but contents of HVA(Homovanillic acid) decreased significantly. These results are ascribable to the suppressed degradation of dopamine induced by selective inhibition of $MAO_B$ by selegiline.
On the other hand, there were no significant changes seen in Ginkgo biloba extract-alone group [Biogenic amines., 4. 351 (1987)], but when given in combination with selegiline, it seemed that the former has further potentiated the increase of dopamine by the latter.

② Measurement of energy metabolism in the cerebral cortex

In ischemia, glycolysis is stimulated under oxygen deficiency and thus, the level of ATP is known to decrease while that of lactates is increased. After inducing ischemia to the brain in rats of both normal and test groups, concentrations of ATP, lactates, pyrubates and glucose were measured and the results are shown in Table 4.

In comparison to normal group, rats of test groups with induced ischemia showed a significant decrease in the level of ATP while a significant increase was observed in the level of lactates in the rat cerebral cortex.

Although no significant changes in these parameters were seen in selegiline-alone group, a significant suppression in the increase of lactate was observed in Ginkgo biloba extract-alone group (p<0.05) in comparison to control group.

Moreover, in the case of selegiline-Ginkgo biloba extract combination group, a significant increase in the level of ATP (p<0.05) was observed together with a very significant decrease in the level of lactates(p<0.001).

These results suggest a possibility that Ginkgo biloba extract has improved a disturbance in energy metabolism due to ischemia through its blood-flow increasing action. Further, Karcher et al reported an increase in the level of ATP in the cerebellum after administration of Ginkgo biloba extract [Naunyn - Schmiedebergs Arch. Pharmacol., 327, 31(1984)] and they also reported changes in energy metabolism in the brain which uptake glucose and deoxyglucose. From these results, it was postulated that the drug may act favorably to suppress damage to the brain and improve memory and learning function.

[Table 4]

| Effects of 3-week treatments with selegiline-alone, Ginkgo biloba extract-alone and selegiline-Ginkgo biloba extract in combination on energy metabolism in the cerebral cortex of ischemic rats. | | | | |
|---|---|---|---|---|
| Drug | ATP (µmol /g wet wt.) | Lactate (µmol/g wet wt.) | Pyruvate (µmol/g wet wt.) | Glucose (µmol/g wet wt.) |
| Normal | 2.30±0.04 | 1.39±0.12 | 0. 08±0.00 | 1.61±0.11 |
| Control | 0.86 ±0.23### | 11.7±0.8### | 0.09 ±0.005 | 1.01 ±0.34 |
| Selegiline | 1.12±0.04 | 5.42±0.15 | 0.10±0.011 | 1.21 ±0.02 |
| Ginkgo Ext. | 1.45±0.12 | 3.21 ±0.11** | 0.10±0.005 | 1.22±0.15 |
| Selegiline[+] Ginkgo Ext. | 2.15±0.15* | 1.29±0.01*** | 0.11±0.009 | 1.51±0.07 |
| Each value represents mean±S. E. (n=6) | | | | |

\* p<0.05

** p<0.01

*** p<0.001: Significantly different from the control.

### p<0. 001; Significantly different from the normal.

(3) Antioxidant effects in partial ischemia and subsequent reperfusion

A damage is known to result in the neuronal cells of the brain when the brain is reperfused after induced ischemia. On the other hand, both selegiline and Ginkgo biloba extract are reported to possess antioxidant effects. In rats of both normal and test groups treated with drugs for 3 weeks, the brain was reperfused after induced partial ischemia for some time and the content of MDA(Malondialdehyde) was measured and enzyme activities of SOD(Superoxide dismutase) and glutathione peroxidase were determined. The results are shown in Table 5.

In comparison to the normal group, there was a significant increase in the level of MDA(Malondialdehyde), which is an indicator for lipid hyperoxidation, in rats of the control group to which a partial ischemia was induced and followed by reperfusion.

In both selegiline-alone or Ginkgo biloba extract-alone groups, no significant changes were observed. But, in contrast, in the case of selegiline-Ginkgo biloba extract combination group, a tendency of decrease in the level of MDA (Malondialdehyde) was observed and significant increases(p<0.05) were observed in the activities of SOD(Superoxide dismutase). which removes superoxide anions and glutathione(GSH) peroxidase, which removes hydrogen peroxide.

These results suggest that antioxidant effects are more potentiated in combined administration of selegiline and Ginkgo biloba extract than in single administration of either drug.

[Table 5]

| Effects of 3-week treatments with selegiline-alone, Ginkgo biloba extract-alone and selegiline-Ginkgo biloba extract in combination on the level of MDA and on activities of SOD and glutathione peroxidase. | | | |
|---|---|---|---|
| Drug Treatment | MDA (nmol/mg protein) | SOD activity (units/mg protein) | GSH peroxidase (units/mg (units/mg protein) |
| Normal | 0.90 ±0.14 | 0.45 ±0.05 | 2.42 ±0.21 |
| Control | 1.86±0.23## | 0.41 ±0. 04 | 1. 98 ±0.14 |
| Selegiline | 1.80±0.22 | 0.42±0.04 | 2.04±0.22 |
| Ginkgo Ext. | 1.69 ±0.08 | 0.40 ±0.03 | 2.28 ±0.25 |
| Selegiline-Ginkgo Ext | 1.50±0.23 | 0.65±0.07* | 3.34±0.37* |
| Each value represents mean±S.E.(n=6) | | | |

\# $p<0.05$; Significantly different from the control.

\#\# $p<0.01$: Significantly different from the normal.

(4) Histopathological examination of the hippocampus in complete ischemia and subsequent reperfusion.

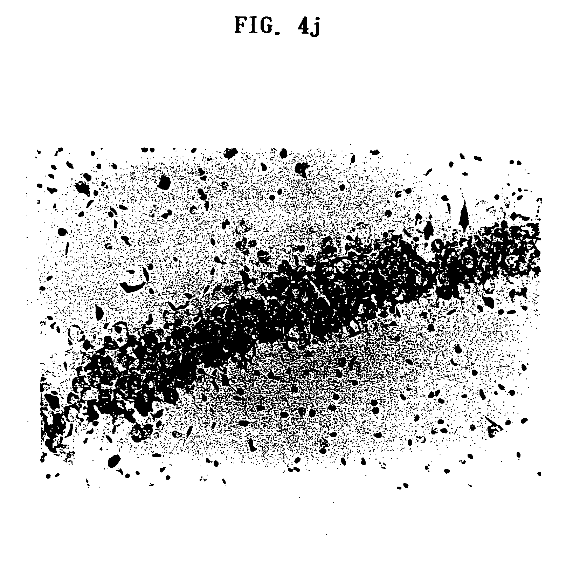

In the cases of complete ischemia and subsequent reperfusion, biochemical changes will appear initially but some morphological changes may result after a few days. After inducing a complete ischemia to the brain and performing subsequent reperfusion for 7 days in gerbils treated with drugs for 3 weeks, microscopic, histopathological examination of the hippocampus was made under cresyl-violet staining and the results are shown in the figures 4a through 4j.

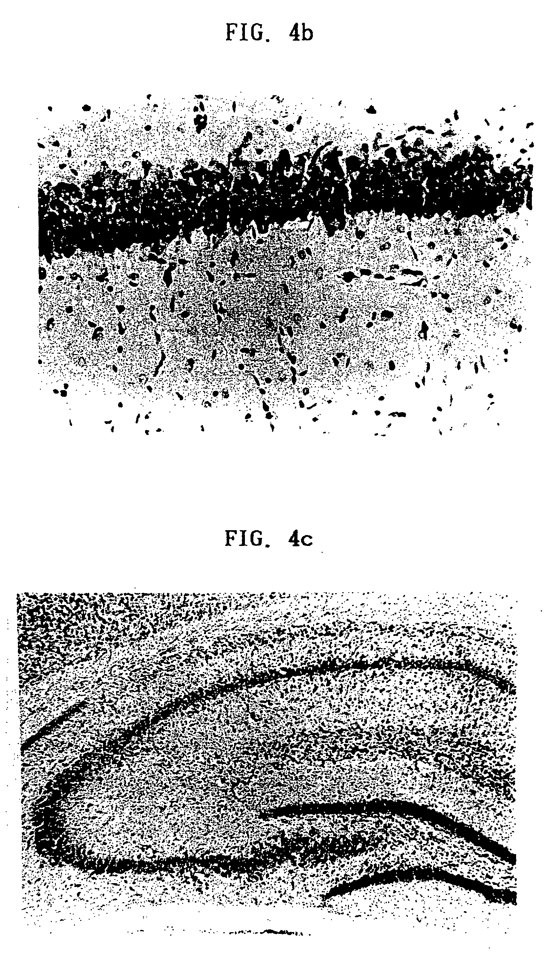

Figure 4a shows a view of the pyramidal cells at CA1 Ammon's horn site of the hippocampus at a lower (×40) magnification while figure 4b shows the same photograph at a higher(×200) magnification ratio.

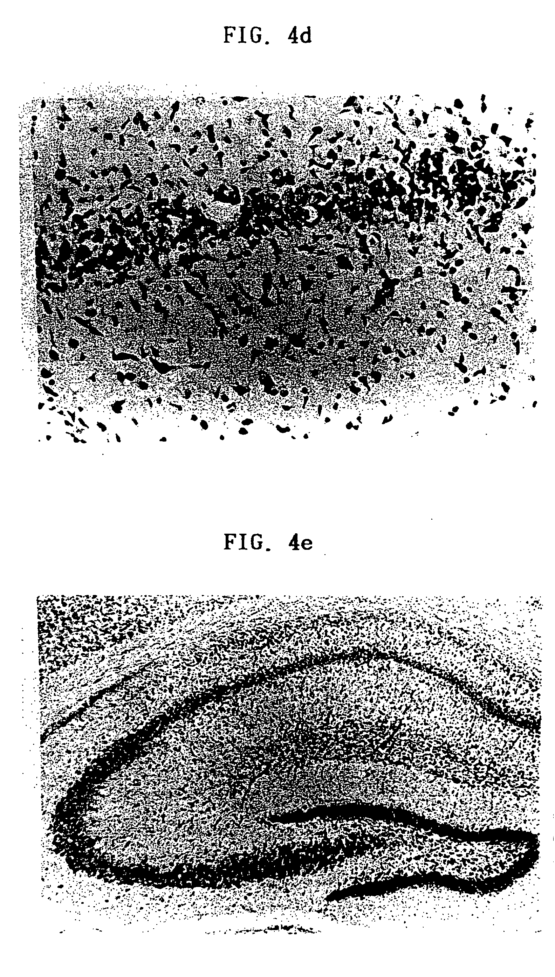

Figure 4c is a view of the pyramidal cells at CA1 Ammon's horn site of the hippocampus of a control rat reperfused for 7 days after an induced complete ischemia by ligation of carotid arteries on both sides of the brain, shown at a lower(×40) magnification while figure 4d shows the same photograph at a higher(X200) magnification ratio, in which most of the pyramidal cells are apparently destroyed or deformed.

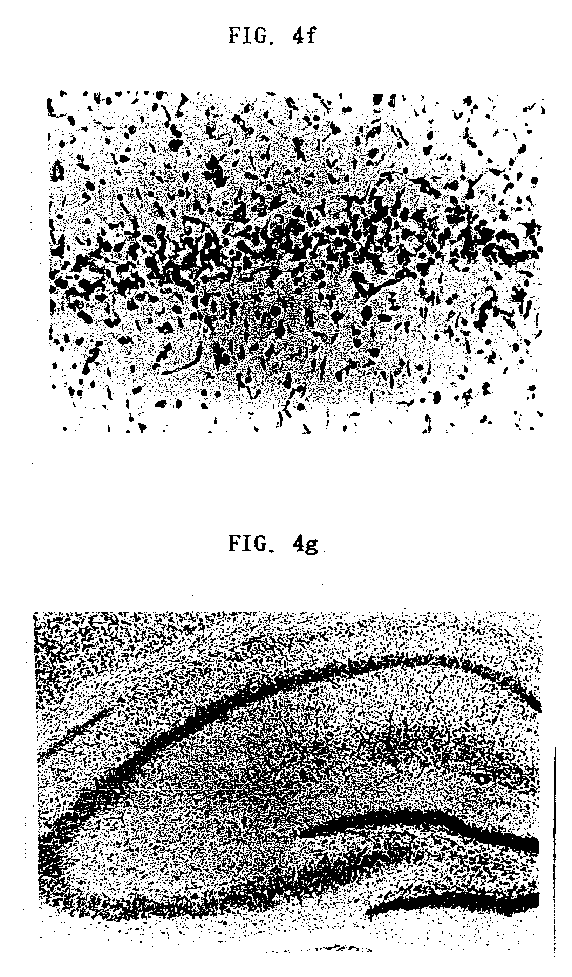

Meanwhile, figure 4e is a view of the pyramidal cells at CA1 Ammon's horn site of the hippocampus of a rat in selegiline-alone group, which however, did not show any effects of significance for protection of the cells in comparison to the control group and figure 4f shows the same photograph at a higher(X200) magnification ratio, in which most of the pyramidal cells are destroyed or deformed.

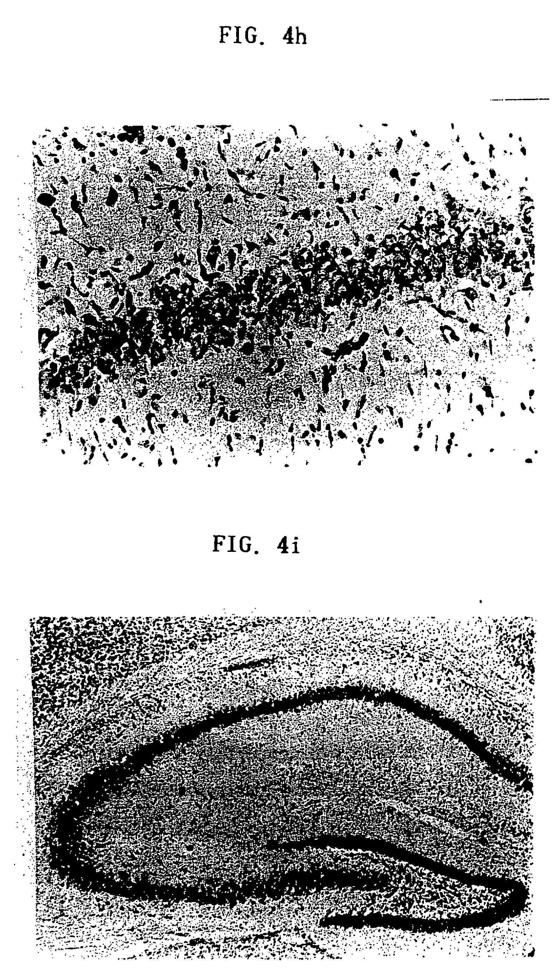

Figure 4g is that of a rat in Ginkgo biloba extract-alone group which shows partially protective effects and figure 4h is a view of the same photograph at a higer(X200) magnification which also demonstrates that destruction of nerve cells are delayed to some extent.

Finally, figure 4i is that of a rat in selegiline-Ginkgo biloba extract combination group in which the pyramidal cells at CA1 Ammon's horn site are shown quite well preserved and the protective effects are more clearly visible than in Ginkgo biloba extract-alone group. Figure 4j is a view of the same photograph, 4i, at a higher(X200) magnification.

These results were also in good harmony with the results of measurement of the density of nerve cells at CA1 site of the hippocampus shown in Table 6 below.

[Table 6]

| Nerve cell density at CA1 site | |
|---|---|
| Drug Treatment | Nerve Cell Density(/mm) |
| Normal | 230±16 |
| Control | 42 ±5 |
| Selegiline | 45±10 |
| Ginkgo Ext | 169 ±10 |
| Selegiline + Ginkgo Ext | 201 ±12 |
| Notes: 1. Each value is mean ± standard error(n=3-4) 2. Image analysis system: Polaroid digital microscopic camera (Optimas version 6.2) | |

4) Conclusion

An attempt was made to achieve enhanced therapeutic effects in the treatment of senile dementia by combined administration of selegiline, a known $MAO_B$ inhibitor which is effective in the treatment of Alzheimer disease by exerting antidepressant effects through selective increase in dopamine level and Ginkgo biloba extract, known to possess PAF antagonism and antioxidant effects and is effective in senile dementia caused by brain ischemia.

Behavioral pharmacological experiments were carried out in rats, using those with memory loss induced by pretreatment with scopolamine as control and other experiments were carried out in rats or gerbil by using those with induced ischemia due to ligation of the carotid arteries on both sides as control. As for test drugs, selegiline at a dose of 2 mg/kg/day or Ginkgo biloba extract at a dose of 48 mg/kg/day was administered either singly or in combination for 3 consecutive weeks by oral route and the effects were compared.

In the behavioral pharmacological experiments, or in the passive avoidance response test and in the maze test, more specifically, significant improvement effects were observed in both selegiline-alone group(p<0.05) and selegiline-Ginkgo biloba extract combi-nation group(p<0.01) in comparison to control group.

In partial ischemia model of rats, dopamine concentration in the cerebral cortex was significantly increased in selegiline-Ginkgo biloba combination group in comparison to control group while those of DOPAC and HVA were decreased. In addition, measurement of energy metabolism in the cerebral cortex demonstrated a decrease in ATP level while lactates were increased.

In the case of ATP level, the combined administration only could bring forth a significant increase(p<0.05) while, in the case of lactates, both Ginkgo biloba extract-alone and the combined administration demonstrated significant decreases. In the case of partial ischemia and subsequent 3-hour reperfusion model, a tendency of suppression of hyperoxidation in the cerebral cortex was observed and activities of SOD and glutathione peroxidase were increased.

On the other hand, the damages caused to the pyramidal cells at CA1 site of the hippocampus of gerbils reperfused after induction of complete ischemia did not show, when examined under cresyl-violet staining, any improvement in selegiline-alone group in comparison to control but some effects of cell-protection were observed in Ginkgo biloba extract-alone group and the protective effects were greater in selegiline-Ginkgo biloba extract combination group.

The results shown above suggest in large that combined use of selegiline and Ginkgo biloba extract can work synergistically in the relief of various symptoms of dementia and protect the neuronal cells by reducing oxidative stress due to ischemia through increased antioxidant action.

(Experiment Example 3)

Single-dose toxicity study for combination product containing selegiline 5 mg and Ginkgo biloba extract 120 mg.

1) Method of experiment

This experiment was carried out in compliance with the methods of single-dose toxicity study described in the Notice No.1998-116 by Korea Food and Drug Administration Office, titled "Standards for Toxicity Studies of Drugs etc.(December 3, 1998)".

Animals: Healthy male(30-35 g in body weight) and female (20-25 g in body weight) ICR-mice bred in the same environmental conditions were used for experiment. Animals were kept in polycarbonate cages placed in a controlled environment with temperature at 23±3 °C and relative humidity at 50±10 %. Sterilized solid animal feeds and purified water were freely supplied.

Reagents and preparation: Selegiline (Lot No.9706005) was supplied by Sanofi S.A., France and Ginkgo biloba extract(Lot No. 708601-1) was Yuyu Industrial Co., Ltd.

Methods of experiment: Single-dose toxicity study on the combination product containing 5 mg of selegiline and 120 mg of Ginkgo biloba extract was carried out. Animals were divided into five dosage groups of 1,000, 2,000, 4,000, 6,000 and 8,000 mg per kg of body weight, respectively, with each group consisting of 10 heads each of both male and female ICR-mice and the drug was given orally at a single dose to each individual animal.

After drug administration, daily observations were made for general conditions and body weight check-ups were made for more than three times until 1 count was made for the death toll after 14 days.

On the basis of drug concentration, death rate and according to Litchfield & Wilcoxon method, the values for $LD_{50}$ were calculated. Further, after 14 days, animals were sacrificed and anatomical examinations were made.

## EP 1 171 146 B1

2) Results and Discussion

To each of 10 male ICR-mice(30-35 g body weight) in each group, the combination product containing 5 mg of selegiline and 120 mg of Ginkgo biloba extract was administered orally at a single dose defined for each group, selecting out of 1,000, 2,000, 4,000, 6,000 and 8,000 mg per kg of body weight, respectively.

In groups of dose of over 4,000 mg/kg, severe reductions in the amount of exercises were observed but it returned to the normal level from the next day and looked similar to the control group by appearance.

Moreover, all death cases were observed one to two days after drug administration and the death counts were 1 at the dose of 2,000 mg/kg, 2 each at the doses of 4,000 mg/kg and 6,000 mg/kg, respectively and 6 at the dose of 8,000 mg/kg.

From these results, the value for $LD_{50}$ in male ICR-mice was calculated at 10,964 mg/kg(5,770-20,831 mg/kg: 95 % confidence limit). If the fact that the drug was administered in 1:24 combination of selegiline and Ginkgo biloba extract, is taken into consideration, this value is equivalent to the simultaneous administration of 438 mg/kg of selegiline and 10,526 mg/kg of Ginkgo biloba extract respectively.

On the other hand, death counts in female mice after 14 days were 2 at the dose of 4,000 mg/kg, 3 at the dose of 6,000 mg/kg and 6 at the dose of 8,000 mg/kg, respectively. From these results, the value for $LD_{50}$ was calculated at 9,332 mg/kg(5,832-14,931 mg/kg: 95 % confidence limit).

If the fact that the drug was administered in 1:24 combination of selegiline and Ginkgo biloba extract, is taken into consideration, this value is equivalent to the simultaneous administration of 373 mg/kg of selegiline and 8,959 mg/kg of Ginkgo biloba extract, respectively.

According to the published literatures, the $LD_{50}$ value for single oral administration of selegiline in mice is 385 mg/kg. Thus, the $LD_{50}$ value for the combination product of selegiline and Ginkgo biloba extract(1:24) was similar to that of single administration of selegiline and it was conceived that the value was not greatly influenced by combination of Ginkgo biloba extract.

Besides, body weight changes were as shown in Table 7. Some reductions in the body weights were observed one day after the drug administration in male mice in the groups of doses of 4,000 mg/kg and 8,000 mg/kg and female mice in the group of dose of 8,000 mg/kg, respectively but the body weights recovered gradually and returned almost to the normal level 7 days after drug administration.

[Table 7]

| Effects of oral administration of combination product selegiline and Ginkgo biloba extract on body weights in mice | | | | | | |
|---|---|---|---|---|---|---|
| Sex | Dose (mg/kg, p.o.) | Days After Treatment | | | | Net body gain (g) weight |
| | | 0 | 1 | 3 | 7 | |
| Male | 1,000 | 34±1 | 33±1 | 33±1 | 34±2 | 0±0 |
| | 2,000 | 32±1 | 31 ±1 | 31±1 | 31±1 | -1±0 |
| | 4,000 | 32±1 | 29±1 | 30±1 | 31±1 | -1±0 |
| | 6,000 | 33±1 | 32±1 | 32±1 | 32±0 | -1±0 |
| | 8,000 | 32±1 | 29±1 | 32±0 | 31 ±1 | -1±0 |
| Female | 1,000 | 23±1 | 23±0 | 23±0 | 24±1 | 1±0 |
| | 2,000 | 23±0 | 23±1 | 24±1 | 24±1 | 1±0 |
| | 4,000 | 24±1 | 23±1 | 23±1 | 23±1 | -1±0 |
| | 6,000 | 25±2 | 25±2 | 24±1 | 24±1 | -1±0 |
| | 8,000 | 23±1 | 20±1 | 21 ±1 | 22±1 | -1±0 |

At 14 days after drug administration, animals were operated on the abdomen and anatomical examinations were made but there were not any remarkable lesions observed in major organs of both male and female mice.

3) Conclusion

The $LD_{50}$ value of a single oral administration of the 1:24 combination product of 5 mg of selegiline and 120 mg of Ginkgo biloba extract in male ICR-mice(30-35 g body weight) was 10,964 mg/kg( 5,770-20,831 mg/kg: 95 % confidence limit) and that for female mice(20-25 g body weight) was 9,332 mg/kg(5,832-14,931 mg/kg:95 % confidence limit).

[Impacts of Invention]

The present invention provides novel composition drugs for treatment of parkinson's disease and dementia, containing selegiline and Ginkgo biloba extract at the ratios between 1:15 and 1:30.
Besides, the composition products of the present invention were stable for more than 24 months expiry in terms of pharmaceutical stability and were proved so safe upon test that any toxicities might be neglected.

**Claims**

1. A pharmaceutical composition for treatment of parkinson's disease and dementia consisting essentially of selegiline and Ginkgo biloba extract compounded at the ratios between 1:15 and 1:30 by weight.

2. The composition according to claim 1, which contains selegiline in an amount of between 2 mg and 5 mg and Ginkgo biloba extract in an amount of between 30 mg and 150 mg per dosage form.

3. The composition according to claims 1 or 2, which contains 5 mg of selegiline hydrochloride and 120 mg of Ginkgo bi loba extract.

4. The composition according to claim 3, which contains 5 mg of selegiline hydrochloride, 120 mg of Ginkgo biloba extract and one or more of pharmaceutically acceptable carriers selected from among a group consisting of excipients, disintegrating agents, binders and lubricating agents.

5. The composition according to claim 4 which is a pharmaceutical dosage form selected from among a group consisting of film-coated tablet, hard gelatin capsule and granule.

**Patentansprüche**

1. Pharmazeutische Zusammensetzung für die Behandlung der Parkinson schen Erkrankung und Demenz, bestehend im Wesentlichen aus Selegilin und Ginko-Biloba-Extrakt, vermischt in Verhältnissen zwischen 1:15 und 1:30 in Gewicht.

2. Zusammensetzung gemäß Anspruch 1, die Selegilin in einer Menge von zwischen 2 mg und 5 mg und Ginko-Biloba-Extrakt in einer Menge von zwischen 30 mg und 150 mg pro Dosisform enthält.

3. Zusammensetzung gemäß Anspruch 1 oder 2, die 5 mg Selegilinhydrochlorid und 120 mg Ginko-Biloba-Extrakt enthält.

4. Zusammensetzung gemäß Anspruch 3, die 5 mg Selegilinhydrochlorid, 120 mg Ginko-Biloba-Extrakt und einen oder mehrere pharmazeutisch annehmbare Träger enthält, ausgewählt aus einer Gruppe, bestehend aus Hilfsstoffen, Desintegrationsmitteln, Bindern und Gleitmitteln.

5. Zusammensetzung gemäß Anspruch 4, bei der es sich um eine pharmazeutische Dosisform handelt, ausgewählt aus einer Gruppe, bestehend aus filmbeschichteten Tabletten, Hartgelatinekapseln und Granulat.

**Revendications**

1. Composition pharmaceutique pour le traitement de la maladie de Parkinson et de la démence constituée essentiellement de sélégiline et d'extrait de Ginkgo biloba formulé aux rapports pondéraux compris entre 1:15 et 1:30.

2. Composition selon la revendication 1, qui contient de la sélégiline à raison de 2 mg à 5 mg et de l'extrait de Ginkgo biloba à raison de 30 mg à 150 mg par forme de dose.

3. Composition selon la revendication 1 ou 2, qui contient 5 mg de chlorhydrate de sélégiline et 120 mg d'extrait de Ginkgo biloba.

**4.** Composition selon la revendication 3, qui contient 5 mg de chlorhydrate de sélégiline, 120 mg d'extrait de Ginkgo biloba et un ou plusieurs des véhicules acceptables sur le plan pharmaceutique choisis dans le groupe formé par les excipients, les agents de désintégration, les liants et les agents lubrifiants.

**5.** Composition selon la revendication 4, qui est une forme de dosage pharmaceutique choisie dans le groupe formé par un comprimé pelliculé, une gélule et un granulé.

FIG. 1

FIG. 2

## FIG. 3

## FIG. 4a

FIG. 4b

FIG. 4c

FIG. 4d

FIG. 4e

FIG. 4f

FIG. 4g

FIG. 4h

FIG. 4i

FIG. 4j